# DEMANDE DE BREVET EUROPEEN

(11) **EP 1 013 248 A1**
(43) Date de publication de la demande: **28.06.2000**
(21) Numéro de dépôt: 99403139.1
(22) Date de dépôt: 14.12.1999
(51) Int. Cl.: A61F 5/37

(54) **Dispositif immobilisateur de l'épaule et du membre supérieur et épaulette utilisable dans ce dispositif**

(30) Priorité: 18.12.1998 FR 9816059
(71) Demandeur: PETERS, 93003 Bobigny Cédex (FR)
(72) Inventeur: Perot, Patrick, 78600 Le Mesnil le Roi (FR)
(74) Mandataire: Michelet, Alain

(57) **Abrégé**

Le dispositif comporte:
(i) un premier ensemble, en soi connu, comprenant au moins une ceinture thoracique (3, 103) et un support d'avant-bras (30, 130),
(ii) une épaulette (2, 102) assurant la contention de l'épaule recouvrant l'épaule en épousant le galbe de celle-ci et se prolongeant vers le bas en avant et en arrière du tronc par un prolongement antérieur (21, 121) et postérieur (20, 120) jusqu'à, respectivement, une extrémité antérieure (23, 123) et postérieure (22, 122), comportant une ou plusieurs bandes du type Velcro® permettant une fixation ajustable et amovible sur la face externe de la ceinture thoracique, respectivement en avant et en arrière du tronc,
caractérisé en ce que l'épaulette se prolonge le long du bras à partir de l'épaule par un prolongement latéral (28, 128) jusqu'à une extrémité latérale (29, 129), les extrémités latérale (29, 129), antérieure (23, 123) et postérieure (22, 122) de l'épaulette (2, 102) étant sensiblement dans le prolongement les unes des autres.

## Description

La présente invention, qui est applicable en orthopédie, a pour objet un dispositif immobilisateur de l'épaule et du membre supérieur. Elle concerne aussi une épaulette utilisable dans ce dispositif.

Pour les traitements des traumatismes, fractures, luxations, entorses ou des suites chirurgicales du membre supérieur, de l'épaule et de la ceinture scapulaire, l'immobilisation garde une place importante.

Depuis le siècle dernier, de nombreux dispositifs ont été proposés à cette fin. On connaît ainsi les bandages de l'illustre professeur DUJARRIER destinés à immobiliser un membre supérieur et l'épaule correspondante d'un patient. D'autres dispositifs se voulant plus simples à mettre en oeuvre et plus efficaces ont ensuite été décrits et utilisés. Parmi ceux-ci, on connaît les dispositifs comportant une bande ou ceinture thoracique enveloppant le tronc du patient.

Par exemple, le document US-3.780.729 propose l'utilisation d'une ceinture thoracique dont toute la face extérieure est recouverte d'un tissu de velours compatible avec un tissu comportant des crochets du type Velcro®. Le terme compatible indique que le tissu de velours et le tissu comportant des crochets du type Velcro® peuvent s'accrocher ou coopérer entre eux, formant ainsi une fixation ou fermeture amovible et réglable. Le tissu de velours comportant des boucles de fils est considéré comme l'élément femelle de la fermeture. Le tissu comportant des crochets est considéré comme l'élément mâle. Cette ceinture présente une certaine élasticité, car la matière dont elle est constituée comporte de la mousse plastique. A une extrémité de la ceinture, sur la face opposée au tissu de velours, est disposée une bande de tissu comportant des crochets du type Velcro®. Cette ceinture entoure le tronc du patient au niveau du thorax. La fermeture type Velcro® permet un réglage et un positionnement aisés de la ceinture thoracique qui est ainsi adaptable à tous les types de conformation de patients. Sur cette ceinture thoracique est adapté au moins un moyen permettant de soutenir l'avant-bras, dit support d'avant bras. Ce moyen de soutien (ou support) est constitué par un manchon ou bracelet comportant sur sa face extérieure un tissu comportant des crochets du type Velcro® permettant ainsi de le fixer dans toutes les positions possibles sur la ceinture.

Dans d'autres immobilisateurs connus, la ceinture thoracique recouvre aussi le bras du membre à immobiliser afin de le plaquer contre le thorax. Comme dans la réalisation décrite précédemment, la ceinture thoracique est recouverte extérieurement d'un tissu de velours compatible avec un tissu comportant des crochets du type Velcro®.

Dans certains dispositifs connus, la ceinture thoracique peut être passée sur un support d'avant bras qui est indépendant et qui peut comporter une ou deux bretelles.

Ces divers dispositifs permettent l'immobilisation du membre supérieur. Cependant, ils n'empêchent pas tous les mouvements de l'épaule et des éléments associés de la ceinture scapulaire. En particulier, ils n'empêchent pas les mouvements verticaux de haut en bas et les mouvements antéro-postérieurs. L'épaule n'est donc pas totalement immobilisée.

On connaît également par US-5,358,470 un immobilisateur d'épaule comportant une simple bande passant sur la clavicule, ladite bande étant fixée sur une bande thoracique comportant un moyen de maintien de l'avant-bras. Ce dispositif est destiné au traitement des fractures de la clavicule et n'est pas adapté aux autres types de traumatismes. En particulier aucune contention du bras n'est assurée.

Ces divers dispositifs ne sont donc pas parfaitement adaptés pour le traitement des fractures d'éléments de la ceinture scapulaire et en particulier de la tête humérale ou du tiers supérieur de l'humérus.

La présente invention propose de résoudre ces problèmes avec un dispositif immobilisateur de l'épaule et du membre supérieur d'un patient, ledit dispositif comportant, en combinaison,
(i) un premier ensemble, en soi connu, comprenant au moins une ceinture thoracique et un support d'avant-bras, ladite ceinture présentant une face interne tournée vers le patient et une face externe opposée à la précédente, la face externe au moins étant compatible avec un tissu comportant des crochets du type Velcro®, et
(ii) une épaulette assurant la contention de l'épaule, ladite épaulette ayant une face interne tournée vers le patient et une face externe opposée à la précédente, ladite épaulette recouvrant l'épaule en épousant le galbe de celle-ci et se prolongeant vers le bas en avant et en arrière du tronc par un prolongement antérieur et un prolongement postérieur jusqu'à, respectivement, une extrémité antérieure et une extrémité postérieure, lesdites extrémités antérieure et postérieure comportant sur leurs faces internes une ou plusieurs bandes d'un tissu comportant des crochets du type Velcro®, permettant leur fixation ajustable et amovible sur la face externe de la ceinture thoracique, respectivement en avant et en arrière du tronc.

Selon l'invention, l'épaulette se prolonge le long du bras à partir de l'épaule par un prolongement latéral jusqu'à une extrémité latérale, les extrémités latérale, antérieure et postérieure de l'épaulette étant sensiblement dans le prolongement les unes des autres.

Dans divers modes de réalisation de l'invention les moyens suivants pouvant être combinés entre eux sont également mis en oeuvre:
L'extrémité latérale de l'épaulette comporte sur sa face interne une ou plusieurs bandes d'un tissu comportant des crochets du type Velcro® permettant sa fixation ajustable et amovible sur la face externe de la ceinture thoracique;
Au moins une des extrémités antérieure et postérieure de l'épaulette s'étend le long de la ceinture thoracique au moins sur une partie de l'hémi-tronc correspondant au coté du membre immobilisé;
Au moins une des extrémités antérieure et postérieure de l'épaulette s'étend le long de la ceinture thoracique au moins sur une partie de l'hémi-tronc correspondant au coté opposé au membre immobilisé;
L'épaulette est réalisée par réunion de deux pièces approximativement triangulaires réunies le long d'un côté recourbé de manière que l'épaulette épouse le galbe de l'épaule;
Les parties antérieure et postérieure de l'épaulette sont symétriques, de sorte que l'épaulette peut être utilisée indifféremment pour l'épaule droite ou gauche;
L'épaulette a une structure lui conférant une certaine élasticité, par exemple en comprenant une mousse de plastique;
L'épaulette comporte sur sa face interne un tissu éponge;
La pièce est approximativement triangulaire droit, les deux bords libres du triangle étant approximativement de même longueur;
La pièce est approximativement triangulaire non droit, les deux bords libres du triangle étant de longueur différente;
Les deux pièces réunies sont identiques;
L'épaulette est formée d'une seule pièce configurée pour épouser le galbe de l'épaule;

Les parties antérieure et postérieure de l'épaulette sont symétriques, de sorte que l'épaulette peut être utilisée indifféremment pour l'épaule droite ou gauche.

L'invention a également pour objet, à titre de moyen constitutif du dispositif immobilisateur décrit précédemment, une épaulette de maintien comportant les caractéristiques ci-dessus indiquées.

L'épaulette de maintien selon l'invention peut être adaptée à divers types connus d'ensembles d'immobilisation, de sorte que les services spécialisés déjà en possession de tels ensembles peuvent réaliser le dispositif objet de la présente invention en combinant ces ensembles à une épaulette proposée séparément. Les personnes qui sont habituées à utiliser de tels ensembles n'ont donc aucune difficulté à manipuler le dispositif immobilisateur selon l'invention.

En raison de sa structure en deux parties, le nouveau dispositif se prête particulièrement bien à des contrôles après la mise en place sur le patient. Il suffit notamment d'enlever l'épaulette ou de soulever une extrémité de l'épaulette pour accéder à l'épaule, sans être obligé de retirer l'ensemble de contention et, ainsi, sans rompre totalement la contention. L'épaule devient alors accessible pour des soins externes, par exemple pour renouveler un pansement, mettre en place une poche de glace ou pour la toilette du patient.

L'invention procure un dispositif utilisable dans la réduction des fractures du tiers supérieur de l'humérus, de la tête humérale et, d'une façon générale pour l'immobilisation et la contention réglables de la ceinture scapulaire.

Le confort du patient est amélioré vis-à-vis des dispositifs connus du genre gilets. La face interne du dispositif de l'invention peut être réalisée en tissu éponge, possédant des propriétés de douceur et d'absorption, alors que les gilets connus réalisés en polyamide sont irritants et même allergisants, de sorte qu'il faut fréquemment interposer un vêtement en coton entre la peau et le gilet.

Egalement, dans le dispositif de l'invention, le coude reste bien dégagé, ce qui facilite la circulation sanguine du bras. Ainsi les problèmes de circulation liés à l'utilisation des dispositifs connus de type gilets sont évités.

L'épaulette constitutive du dispositif de l'invention peut être utilisée indifféremment du côté gauche ou du côté droit, car, comme on le préfère, elle est réalisée de manière symétrique à l'avant et à l'arrière. Il suffit, en principe, de disposer de deux tailles d'épaulette, une pour enfant et l'autre pour adulte.

De même dans le cas d'une pièce approximativement triangulaire dont les deux côtés libres ont des longueurs différentes, l'utilisateur dispose d'une épaulette dont la courbure au niveau de la réunion des deux pièces est asymétrique. Il est ainsi possible suivant le sens d'insertion de l'épaulette d'obtenir un ajustement parfait sur une épaule forte ou une épaule fine. Ce type d'épaulette permet donc un positionnement parfait à droite ou à gauche aussi bien pour une épaule forte qu'une épaule fine.

Par contre si la pièce présente deux côtés libres de longueurs sensiblement identiques, cet ajustement optimum épaule forte/épaule fine ne sera pas possible et l'on disposera de plusieurs modèles ou on réalisera un modèle moyen, l'élasticité du matériau permettant d'obtenir un ajustement suffisant. Ce dernier type d'épaulette permet également un positionnement à droite ou à gauche.

Enfin, le dispositif de l'invention offre une souplesse inégalée dans le traitement des traumatismes de l'épaule. En effet, grâce à sa constitution, la contention est réglable au cours de la convalescence du patient.

Dans un cas typique de fracture de l'extrémité supérieure de l'humérus, la ceinture thoracique et l'épaulette seront mis en place pendant une dizaine de jours pour une contention et immobilisation maximales permettant au cal osseux de se mettre en place. Au cours de cette période des interventions de soins, toilettage ou autres seront toujours possibles sans rompre totalement la contention en désolidarisant en totalité ou partie seulement l'épaulette.

Dans un deuxième temps, pendant une dizaine de jours, l'épaulette sera retirée, la bande thoracique restant en place. La rééducation pourra être mise en oeuvre.

Finalement, dans un troisième temps, pendant une dizaine de jours, une simple écharpe de soutien sera mise en oeuvre.

Une telle souplesse de mise en oeuvre du traitement est impossible avec les dispositifs classiques et en particulier les Dujarriers ou les gilets.

La présente invention sera illustrée sans être aucunement limitée par la description d'exemples de réalisation faite en regard des dessins annexés sur lesquels:

Fig. 1 représente, vu de face, un dispositif d'immobilisation avec épaulette.

Fig. 2 représente, également vue de face, une variante de réalisation de dispositif d'immobilisation avec épaulette.

Fig. 3 est une vue en perspective de la face intérieure d'une épaulette représentée séparément et non montée.

Fig. 4 est une vue en perspective de la face extérieure de l'épaulette de la Fig. 3.

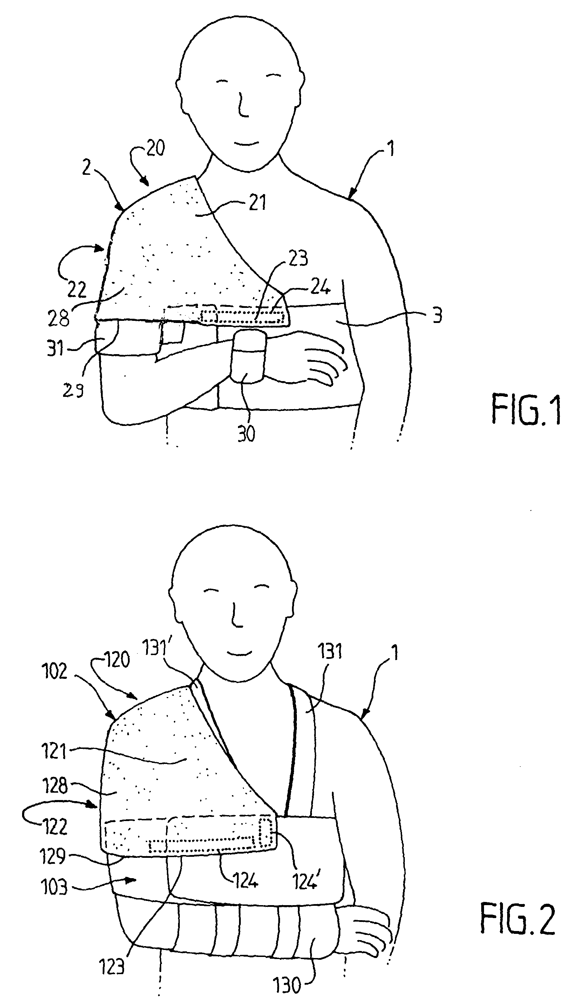

Sur la Figure 1, un patient 1 est porteur d'un ensemble d'immobilisation de type connu, tel que celui décrit dans US-A-3.780.720 avec une ceinture thoracique 3 et un support d'avant-bras 30. La face extérieure de la ceinture thoracique est recouverte d'un tissu du type velours compatible avec un tissu comportant des crochets du type Velcro®. Le support d'avant-bras est un bracelet 30 qui comporte extérieurement un tissu comportant des crochets du type Velcro®. Le bracelet 30 peut ainsi être fixé d'une manière amovible et réglable en position le long de la ceinture 3. Par ailleurs un brassard 31 passé autour du bras comporte extérieurement un tissu comportant des crochets du type Velcro®. Le brassard peut donc être fixé d'une manière amovible et réglable en position le long de la ceinture 3 par une fermeture du type Velcro®.

Selon l'invention, le dispositif immobilisateur comprend une épaulette 2 qui est placée sur l'épaule du côté du membre à immobiliser et épouse le galbe de l'épaule. Les prolongements antérieur 21 et postérieur 20 et latéral 28 se terminent par, respectivement, des extrémités antérieure 23, postérieure 22 et latérale 29. Le prolongement postérieur 20 et son extrémité 22 qui se trouvent dans le dos du patient, ne sont pas visibles sur la figure 1. Aux deux extrémités 22, 23 des prolongements 20, 21, sont disposées, sur la face intérieure de l'épaulette, des bandes de tissu comportant des crochet du type Velcro®. Dans le cadre de l'invention, les bandes peuvent se poursuivre sur le prolongement latéral 28. Sur la face extérieure de l'épaulette 2, des coutures 24 sont visibles, qui correspondent aux bandes de tissu type « Velcro » internes. Cependant, dans d'autres modes de réalisation, les bandes de tissu type « Velcro » pourront être fixées sur la face intérieure de l'épaulette par tout moyen connu, par exemple par collage ou soudure. Chacune des extrémités 22, 23 peut ainsi être fixée sur la ceinture thoracique d'une manière amovible et réglable grâce à une fermeture du type Velcro®. La contention de l'épaule et des éléments corporels associés peut donc être réglée à volonté en intensité comme en direction. Le terme élément corporel associé désigne notamment la clavicule ou l'omoplate. Ainsi on peut à volonté placer et immobiliser, par exemple, l'épaule vers l'avant ou l'arrière suivant l'importance de la traction exercée sur l'épaule par le prolongement antérieur ou postérieur. Par ailleurs, la fixation étant amovible aussi bien antérieurement que postérieurement, le prolongement correspondant et/ou l'épaulette peuvent être soit simplement soulevés dans le cas où une seule extrémité est détachée, soit être retirés. Ces manoeuvres permettent de dégager l'épaule pour inspection, traitement ou lavage, sans pour autant compromettre ou supprimer l'immobilisation prodiguée par le dispositif à ceinture thoracique et support d'avant bras.

Sur la Figure 2, un patient 1 est porteur d'un autre dispositif immobilisateur bras-épaule de type connu, dans lequel une ceinture thoracique 103 recouvre aussi au moins une partie du bras afin de le plaquer contre le thorax. Ce dispositif comporte un support indépendant d'avant-bras 130 muni de deux bretelles 131, 131'. Selon l'invention, une épaulette 102 est placée sur l'épaule du patient. L'épaulette comporte comme dans le premier mode de réalisation des prolongements antérieur 121, postérieur 120 et latéral 128 sur la face externe du bras jusqu'à une extrémité latérale 129. Les trois prolongements antérieur 121, latéral 128 et postérieur 120 sont contigus. Les trois extrémités antérieure 123, latérale 129 et postérieure 122 sont sensiblement dans la continuité et le prolongement les unes des autres. Grâce à une telle disposition, toutes les extrémités 123, 129, 122 sont situées sur la ceinture thoracique 103. Le long de ces extrémités sur la face interne de l'épaulette qui est au contact de la ceinture thoracique, sont fixées des bandes de tissu comportant des crochets du type Velcro®, permettant ainsi un accrochage et une fermeture amovibles et réglables de l'épaulette 102 sur la face externe de la ceinture thoracique 103. Sur la face extérieure de l'épaulette 102, des coutures 124, 124' sont visibles, qui correspondent aux bandes de tissu de type « Velcro ». Cependant, dans d'autres modes de réalisation, les bandes de tissu de type « Velcro » peuvent être fixées sur la face intérieure de l'épaulette par tout moyen connu, par exemple par collage ou soudure.

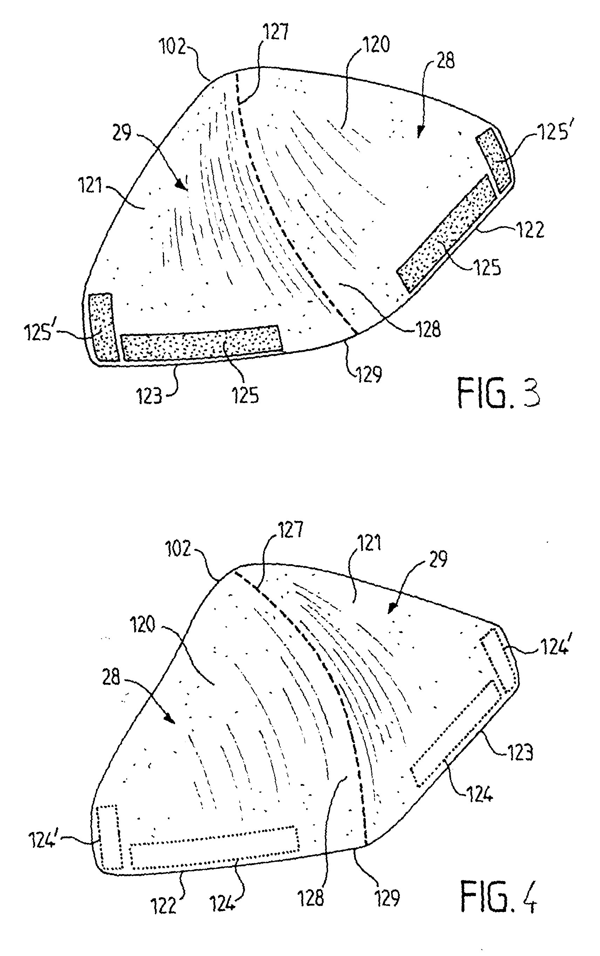

La forme et/ou la disposition et/ou la longueur des bandes de tissu comportant des crochets du type Velcro® peuvent être quelconques, la seule condition étant que la fermeture de l'épaulette 2, 102 sur la ceinture 3, 103 soit assurée. Dans un mode de réalisation préféré, les bandes de tissu comportant des crochets du type Velcro® sont placées au voisinage et le long des deux extrémités antérieure 23,123 et postérieure 22,122 de l'épaulette 2,102. La largeur de la ou des bandes de tissu comportant des crochets sera fonction de l'importance de la résistance recherchée de la fixation. On peut aussi prévoir, afin d'améliorer la résistance de la fermeture, une ou plusieurs bandes de tissu comportant des crochets du type Velcro® disposées le long du bord thoracique et/ou dorsal du prolongement antérieur et/ou postérieur comme on le voit sur la figure 3, en 125'.

La Figure 3 est une vue en perspective de la face intérieure destinée à être au contact du patient, d'une épaulette selon l'invention. Cette épaulette est représentée, non installée sur un patient. Elle est sensiblement symétrique et est préférentiellement réalisée par réunion de deux pièces identiques 28, 29 bord à bord le long d'une ligne 127, la réunion étant par exemple faite par couture des deux pièces. Chacune des deux pièces 28, 29 a une forme approximativement triangulaire. Chacun des deux bords destinés à être cousus ensembles le long de la ligne 127, est recourbé afin que l'épaulette épouse le galbe de l'épaule. Des bandes 125, 125' de tissu comportant des crochets du type Velcro® sont disposées le long des extrémités antérieure 123 et postérieure 122 de l'épaulette 102. Dans cette forme de réalisation, l'extrémité latérale 129 de l'épaulette est dépourvue de tissu d'accrochage type Velcro®, cependant dans d'autres formes, une bande de tissu d'accrochage peut être disposée tout le long des extrémités antérieure 123, latérale 129 et postérieure 122 de l'épaulette .

La Figure 4 est une vue en perspective de la face extérieure opposée à la face intérieure de l'épaulette de la figure 3. Cette épaulette est représentée non installée sur un patient. Une ligne de couture 127 correspondant à la réunion des deux pièces 28, 29 est visible dans ce mode préféré de réalisation. Cependant il est aussi envisagé que les deux pièces soient réunies par un autre moyen, soudure ou collage par exemple. Il est aussi envisagé que l'épaulette soit réalisée d'une seule pièce tout en présentant une forme lui permettant d'épouser le galbe de l'épaule. Des coutures 124, 124' sont visibles sur cette face extérieure de l'épaulette 102, qui correspondent aux bandes de tissu de type « Velcro ». Cependant, dans d'autres modes de réalisation, les bandes de tissu de type « Velcro » peuvent être fixées sur la face intérieure de l'épaulette par tout moyen connu, par exemple par collage ou soudure.

Dans le cas où chacune des pièces triangulaires présente deux bords libres de longueurs différente permettant ainsi par inversion l'adaptation à une épaule fine ou forte, on dispose d'évidence les bandes de tissu de type « Velcro », le long des deux bords libres afin de permettre cette inversion et l'accrochage sur les ceintures 3, 103.

L'épaulette comporte préférentiellement un tissu éponge sur la face destinée à être au contact du patient. De la mousse de préférence à alvéoles ouverts peut être utilisée dans l'épaisseur de l'épaulette. L'épaulette peut d'une façon générale être réalisée de telle façon que l'air puisse passer à travers, par exemple en utilisant de la mousse à pores ouverts et/ou en pratiquant des ouvertures dans un ou plusieurs des tissus constitutifs. L'épaulette présente avantageusement une certaine élasticité afin de pouvoir s'adapter à divers galbes d'épaule.

De par sa construction l'épaulette est avantageusement utilisable aussi bien pour une épaule droite que gauche, les prolongements et extrémités antérieurs et postérieurs s'inversant alors. Les termes antérieur et postérieur sont donc donnés à titre purement illustratif et pour faciliter la compréhension de l'invention. Dans un mode de réalisation préféré, l'épaulette 2, 102 est sensiblement symétrique et en position d'utilisation les extrémités antérieure 23, 123 et postérieure 22, 122 sont situées transversalement et en hauteur approximativement dans la même position respectivement en avant ou en arrière du tronc le long de la ceinture thoracique 3, 103. Les extrémités antérieure et postérieure peuvent atteindre, sans le dépasser, le creux axillaire du coté opposé au membre immobilisé. Cependant il est aussi envisagé dans le cadre de l'invention que l'épaulette soit asymétrique, une des extrémités antérieure ou postérieure s'étendant davantage transversalement et/ou en hauteur par rapport à l'autre le long de la ceinture thoracique.

Les exemples de réalisation ci-dessus décrits ne sauraient limiter la portée de l'invention.

## Revendications

1. Dispositif immobilisateur de l'épaule et du membre supérieur d'un patient (1), ledit dispositif comportant, en combinaison,
(i) un premier ensemble, en soi connu, comprenant au moins une ceinture thoracique (3, 103) et un support d'avant-bras (30, 130), ladite ceinture présentant une face interne tournée vers le patient et une face externe opposée à la précédente, la face externe au moins étant compatible avec un tissu comportant des crochets du type Velcro® et
(ii) une épaulette (2, 102) assurant la contention de l'épaule, ladite épaulette ayant une face interne tournée vers le patient et une face externe opposée à la précédente, ladite épaulette recouvrant l'épaule en épousant le galbe de celle-ci et se prolongeant vers le bas en avant et en arrière du tronc par un prolongement antérieur (21, 121) et un prolongement postérieur (20, 120) jusqu'à, respectivement, une extrémité antérieure (23, 123) et une extrémité postérieure (22, 122), lesdites extrémités antérieure et postérieure comportant sur leurs faces internes une ou plusieurs bandes d'un tissu comportant des crochets du type Velcro® permettant leur fixation ajustable et amovible sur la face externe de la ceinture thoracique, respectivement en avant et en arrière du tronc,
caractérisé en ce que l'épaulette se prolonge le long du bras à partir de l'épaule par un prolongement latéral (28, 128) jusqu'à une extrémité latérale (29, 129), les extrémités latérale (29, 129), antérieure (23, 123) et postérieure (22, 122) de l'épaulette (2, 102) étant sensiblement dans le prolongement les unes des autres.

2. Dispositif selon la revendication 1, caractérisé en ce que l'extrémité latérale (29, 129) de l'épaulette comporte sur sa face interne une ou plusieurs bandes d'un tissu comportant des crochets du type Velcro® permettant sa fixation ajustable et amovible sur la face externe de la ceinture thoracique (3, 103).

3. Dispositif selon la revendication 1 ou 2, caractérisé en ce que au moins une des extrémités antérieure (23, 123) et postérieure (22, 122) de l'épaulette (2, 102) s'étend le long de la ceinture thoracique (3, 103) au moins sur une partie de l'hémi-tronc correspondant au coté du membre immobilisé.

4. Dispositif selon l'une quelconque des revendications précédentes, caractérisé en ce que au moins une des extrémités antérieure (23, 123) et postérieure (22, 122) de l'épaulette (2, 102) s'étend le long de la ceinture thoracique (3, 103) au moins sur une partie de l'hémi-tronc correspondant au coté opposé au membre immobilisé.

5. Dispositif selon l'une quelconque des revendications précédentes, caractérisé en ce que l'épaulette (2, 102) est réalisée par réunion de deux pièces approximativement triangulaires réunies le long d'un côté recourbé (127) de manière que l'épaulette (2, 102) épouse le galbe de l'épaule.

6. Dispositif selon l'une quelconque des revendications précédentes, caractérisé en ce que les parties antérieure (29) et postérieure (28) de l'épaulette (102) sont symétriques, de sorte que l'épaulette peut être utilisée indifféremment pour l'épaule droite ou gauche.

7. Dispositif selon l'une quelconque des revendications précédentes, caractérisé en ce que l'épaulette a une structure lui conférant une certaine élasticité, par exemple en comprenant une mousse de plastique.

8. Dispositif selon l'une quelconque des revendications précédentes, caractérisé en ce que l'épaulette comporte sur sa face interne un tissu éponge.

9. Elément de contention de l'épaule d'un patient destiné à coopérer avec un ensemble, comprenant au moins une ceinture thoracique (3, 103) et un support d'avant-bras (30, 130), ladite ceinture présentant une face interne tournée vers le patient et une face externe opposée à la précédente, la face externe au moins étant compatible avec un tissu comportant des crochets du type Velcro® et ledit élément étant une épaulette selon l'une quelconque des revendications précédentes.
